(19) Europäisches Patentamt
European Patent Office
Office européen des brevets

(11) Veröffentlichungsnummer: **0 040 581**
**B1**

(12) **EUROPÄISCHE PATENTSCHRIFT**

(45) Veröffentlichungstag der Patentschrift: **11.07.84**

(21) Anmeldenummer: **81810176.8**

(22) Anmeldetag: **07.05.81**

(51) Int. Cl.³: **C 07 C 69/73**, C 07 D 307/12, C 07 D 319/12, C 07 C 13/19, C 07 C 121/70, C 07 C 121/38, C 07 C 49/217, C 07 C 103/76, D 06 L 3/12, C 07 C 67/347, C 07 C 2/86

(54) **Verfahren zur Herstellung von Vinylstilbenverbindungen.**

(30) Priorität: **13.05.80 CH 3730/80**
**05.03.81 CH 1474/81**

(43) Veröffentlichungstag der Anmeldung: **25.11.81 Patentblatt 81/47**

(45) Bekanntmachung des Hinweises auf die Patenterteilung: **11.07.84 Patentblatt 84/28**

(84) Benannte Vertragsstaaten: **CH DE FR GB IT LI**

(56) Entgegenhaltungen:
**DE - A - 2 041 563**
**DE - A - 2 059 291**
**US - A - 3 527 794**
**US - A - 4 108 887**

**CHEMICAL ABSTRACTS, Band 92, Nr. 13, 31. März 1980, Seite 593, Zusammenfassung 110202z Columbus, Ohio, USA A. BIAVATI et al. "Palladium or nickel-catalyzed benzoylation and phenylation of methyl acrylate"**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Bellus, Daniel, Dr., Unterm Schellenberg 81, CH-4125 Riehen (CH)**
Erfinder: **Blaser, Hans-Ulrich, Dr., Brücklismattstrasse 18, CH-4107 Ettingen (CH)**
Erfinder: **Kabas, Guglielmo, Dr., Im Egg 36, CH-4147 Aesch (CH)**
Erfinder: **Reinehr, Dieter, Dr., Wolfsheule 10, D-7842 Kandern (DE)**
Erfinder: **Spencer, Alwyn, Dr., Schützengraben 15, CH-4051 Basel (CH)**
Erfinder: **Weber, Kurt, Dr., Rennweg 98, CH-4052 Basel (CH)**

**EP 0 040 581 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Vinylstilbenverbindungen sowie neue Vinylstilbenverbindungen und deren Verwendung zum optischen Aufhellen von organischen Materialien.

Derartige Vinylstilbenverbindungen als optische Aufheller und verschiedene Verfahren zu deren Herstellung sind in der US-PS Nr. 4108887 beschrieben.

Aus der US-PS Nr. 3922299 ist bekannt, dass man vinylisch oder allylisch substituierte organische Verbindungen, besonders Zimtsäure und Zimtsäureester, durch katalytische Umsetzung von entsprechenden Halogeniden mit aktivierten Olefinen, wie Acrylsäuremethylester, in Gegenwart von tertiären Aminen herstellen kann. Als Katalysatoren werden bevorzugt Gemische von Palladiumacetat und Triphenylphosphin oder Tri(orthotolyl)phosphin verwendet.

Die Umsetzung kann auch so vorgenommen werden, dass man zuerst aus dem Halogenid und dem Katalysatorsystem einen Komplex bildet und diesen anschliessend in Gegenwart eines tertiären Amins mit dem Olefin reagieren lässt. Andererseits ist bekannt, dass die Umsetzung von Benzoylchlorid mit Acrylsäuremethylester in Gegenwart stöchiometrischer Mengen eines Nickel (O)katalysators bei Nachbehandlung des Reaktionsgemisches mit Jod in Methanol zur Bildung von trans-3-Benzoylacrylsäuremethylester führt. Als Nebenprodukt fällt dabei Zimtsäuremethylester an. Durch Umsetzung eines Komplexes aus Benzoylpalladiumchlorid und Triphenylphosphin mit Acrylsäuremethylester bei 70-85° C in Gegenwart von Triäthylamin erhält man Zimtsäuremethylester als Hauptprodukt und Benzoylacrylsäuremethylester als Nebenprodukt. Werden als Palladium und das Triphenylphosphin nur in katalytischen Mengen eingesetzt, so verschiebt sich das Reaktionsgleichgewicht zu Gunsten der Bildung des Benzoylacrylsäuremethylesters (Gewichtsverhältnis von Benzoylacrylsäuremethylester/Zimtsäuremethylester = ca. 8,3:1) (vgl. „Transition Met. Chem.", *2*, 270 [1977] und *4*, 398 [1979]). Schliesslich ist aus „Synthesis", 777 (1977) bekannt, dass sich aromatische Säurehalogenide mit Alkinen unter Pd-Katalyse ohne Decarbonylierung zu Alkinylketonen umsetzen lassen.

Es wurde nun gefunden, dass man Vinylstilbenverbindungen der Formel I:

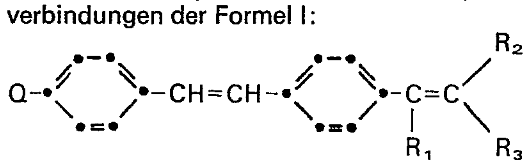 (I)

worin Q für die Gruppierung −COX oder

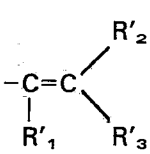 steht, und $R_1$ und $R'_1$ unabhängig voneinander Wasserstoff, Cyano, Alkyl oder eine nicht chromophore veresterte Carboxylgruppe, $R_2$ und $R'_2$ unabhänging voneinander Wasserstoff, gegebenenfalls nicht chromophor substituiertes Alkyl oder Alkenyl oder eine nicht chromophoren Substituenten zweiter Ordnung, $R_3$ und $R'_3$ unabhängig voneinander Wasserstoff, Cyano oder gegebenenfalls nicht chromophor substituiertes Alkyl oder Alkenyl, und X Chlor, Brom oder Jod bedeuten oder $R_1$ zusammen mit $R_3$ bzw. $R'_1$ zusammen mit $R'_3$ eine Alkylenbrücke darstellen, wobei höchstens eines der Symbole $R_2$ und $R_3$ und höchstens eines der Symbole $R'_2$ und $R'_3$ Wasserstoff sind, und wobei, wenn Q für −COX steht, $R_1$ und $R_3$ Wasserstoff und $R_2$ Carbonsäure-$C_{1-4}$-alkylester oder Cyano bedeuten, dadurch herstellen kann, dass man 1 mol-Eq einer Verbindung der Formel II:

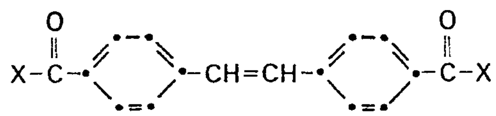 (II)

worin X die oben angegebene Bedeutung hat, in Gegenwart einer Base und unter Zusatz von Palladiumverbindungen, die unter den Reaktionsbedingungen phosphorfreie labile Palladium(O)verbindungen bilden, als Katalysator mit 1 mol-Eq einer Verbindung der Formel III oder mit je 1 mol-Eq der Verbindungen der Formeln III und IV:

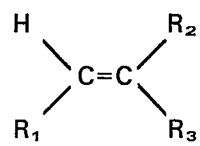 (III) 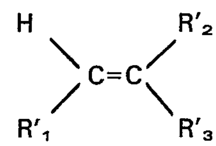 (IV)

worin $R_1$, $R_2$ und $R_3$ bzw. $R'_1$, $R'_2$ und $R'_3$ die oben angegebene Bedeutung haben, umsetzt.

Nach dem erfindungsgemässen Verfahren lassen sich die Verbindungen der Formel I auf einfache wirtschaftliche Weise und unter Verwendung von leicht zugänglichen Ausgangsprodukten herstellen. Dabei ist es überraschend, dass die Reaktion selektiv unter Decarbonylierung der Säurehalogenide der Formel II verläuft.

Die in den Verbindungen der Formel I vorkommenden Substituenten sind im allgemeinen solche, wie sie im Gebiet der optischen Aufheller üblich sind.

Alkylgruppen $R_1$, $R'_1$, $R_2$, $R'_2$, $R_3$ und $R'_3$ können geradkettig oder verzweigt sein und weisen bevorzugt 1 bis 6 und insbesondere 1 oder 2 C-Atome auf. Alkenylgruppen $R_2$, $R'_2$, $R_3$ und $R'_3$ enthalten vorteilhaft 2 bis 4, vorzugsweise 3 C-Atome. Als Beispiele derartiger Alkyl- und Alkenylgruppen seien erwähnt: die Methyl-, Äthyl-, n-Propyl-, Isopropyl-, n-Butyl-, tert.-Butyl-, n-Pentyl-, n-Hexyl-, Vinyl- und Allylgruppe.

Als nicht chromophore Substituenten an definitionsgemässen Alkyl- oder Alkenylgruppen kommen z.B. Alkoxy-, Alkoxyalkoxy-, Dialkylamino- oder Alkoxycarbonylgruppen, ferner Halogenatome, wie Fluor oder Chlor, oder gegebenenfalls nicht chromophor substituiertes Phenyl in Betracht, z.B. gegebenenfalls durch Alkyl, Alkoxy, Halogen, Cyano, eine Carbonsäureester- oder

-amidgruppe oder durch einen Carbonsäureacylrest substituiertes Phenyl. Alkoxy- oder Alkylteile in den genannten Substituenten enthalten vorzugsweise 1 bis 4 und besonders 1 oder 2 C-Atome. Alkenylgruppen $R_2$, $R'_2$, $R_3$ und $R'_3$ sind bevorzugt unsubstituiert. Alkoxyalkoxysubstituenten weisen mit Vorteil 2 bis 6 und insbesondere 4 bis 6 C-Atome auf. Alkylenbrükkenglieder in Alkoxyalkoxysubstituenten sind vorteilhaft Äthylen, Propylen-1,2, Butylen-1,2 oder Butylen-2,3, wovon jedoch Äthylen bevorzugt ist.

Stellen $R_1$ und $R_3$ bzw. $R'_1$ und $R'_3$ zusammen Alkylen dar, so handelt es sich z.B. um die Propylen-1,3- oder Butylen-1,4-Gruppe.

Nicht chromophore Substituenten zweiter Ordnung sind elektronenanziehende Substituenten, die den Verbindungen der Formel I keine Farbe verleihen, was deren Verwendung als optische Aufheller beeinträchtigen würde. Als Beispiele seinen genannt: Acylreste organischer, nicht chromophorer Carbonsäuren, die Cyano- und Trifluormethylgruppe, gegebenenfalls substituiertes Phenylalkyl, Carbonsäureester- und -amidgruppen. Die im Molekül vorkommenden Estergruppen, besonders die nicht chromophoren veresterten Carboxylgruppen sind z.B. gegebenenfalls alkylsubstituierte Carbonsäurecycloalkylestergruppen oder Carbonsäurealkylestergruppen, wobei das Alkyl durch Alkoxy- oder Alkoxyalkoxygruppen der obenerwähnten Art substituiert sein kann; ferner gegebenenfalls substituierte Carbonsäurephenylester- oder -phenylalkylestergruppen, wobei das Phenyl wie oben angegeben substituiert sein kann.

Carbonsäureamidgruppen können substituiert oder vorzugsweise unsubstituiert sein. Als Substituenten kommen z.B. Alkyl-, Alkoxyalkyl-, Dialkylaminoalkyl-, Cycloalkyl- und gegebenenfalls substituierte Phenyl- oder Phenylalkylgruppen in Betracht.

Bevorzugt ist die Herstellung von Verbindungen der Formel I, worin Q für die Gruppierung

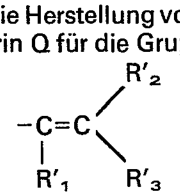

steht, und worin $R_1$ und $R'_1$ unabhängig voneinander Wasserstoff, $-CN$, $C_{1-6}$-Alkyl oder $-COOR'''$, bevorzugt Wasserstoff, $-CN$ oder $C_{1-4}$-Alkyl und insbesondere Wasserstoff oder Methyl, $R_2$ und $R'_2$ unabhängig voneinander Wasserstoff oder gegebenenfalls durch $C_{1-4}$-Alkoxy oder $C_{2-5}$-Alkoxycarbonyl substituiertes $C_{1-6}$-Alkyl, $C_{2-4}$-Alkenyl, $-CN$, $-CF_3$ oder eine Gruppe:

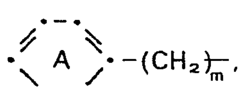

$-CO-R$, $-CO-NR'R''$ oder $-COOR'''$

mit Vorteil $C_{2-4}$-Alkenyl und vor allem $-CN$, $-CONR'R''$, $-COOR'''$ oder $-CO-R$, insbesondere $-CN$ oder $-COOR'''$, und $R_3$ und $R'_3$ unabhängig voneinander Wasserstoff, $-CN$ oder gegebenenfalls durch $C_{1-4}$-Akoxy oder $C_{2-5}$-Alkoxycarbonyl substituiertes $C_{1-6}$-Alkyl, besonders Wasserstoff, $-CN$ oder $C_{1-4}$-Alkyl und vorteilhaft Methyl und ganz besonders bevorzugt Wasserstoff oder $R_1$ und $R_3$ bzw. $R'_1$ und $R'_3$ zusammen $-(CH_2)_3$ oder $-(CH_2)_4$ darstellen, wobei höchstens eines von $R_2$ und $R_3$ und höchstens eines von $R'_2$ und $R'_3$ Wasserstoff sind, R Methyl, gegebenenfalls durch $C_{1-4}$-Alkoxy, $C_{4-6}$-Alkoxyalkoxy substituiertes $C_{2-6}$-Alkyl, einen Rest der Formel:

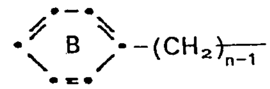

oder Naphthyl, besonders Methyl, gegebenenfalls durch $C_{1-4}$-Alkoxy oder $C_{4-6}$-Alkoxyalkoxy substituiertes $C_{2-6}$-Alkyl, den Rest der Formel:

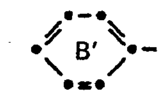

oder Naphthyl und ganz besonders gegebenenfalls durch Chlor, Methyl oder Methoxy monosubstituiertes Phenyl, unsubstituiertes $C_{1-6}$-Alkyl, vorzugsweise $C_{1-4}$-Alkyl und vor allem Methyl, R' Wasserstoff, Methyl, gegebenenfalls durch $C_{1-4}$-Alkoxy, $C_{4-6}$-Alkoxyalkoxy oder Di-($C_{1-4}$-alkyl) amino substituiertes $C_{2-6}$-Alkyl, gegebenenfalls methyl- oder äthylsubstituiertes Cyclohexyl oder einen Rest der Formel:

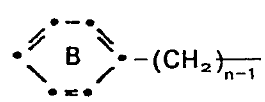

besonders Methyl, R'' Wasserstoff, Methyl oder gegebenenfalls durch $C_{1-4}$-Alkoxy oder $C_{4-6}$-Alkoxyalkoxy substituiertes $C_{2-6}$-Alkyl, besonders Methyl, R''' Methyl, gegebenenfalls durch $C_{1-4}$-Alkoxy oder $C_{3-6}$-Alkoxyalkoxy substituiertes $C_{2-6}$-Alkyl, gegebenenfalls methyl- und/oder äthylsubstituiertes Cyclohexyl oder einen Rest der Formel:

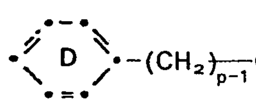 oder 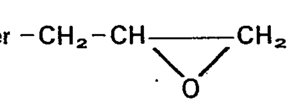

und besonders Methyl oder gegebenenfalls durch $C_{1-4}$-Alkoxy oder $C_{3-6}$-Alkoxyalkoxy substituiertes $C_{2-6}$-Alkyl, insbesondere Alkyl mit 1 bis 6 und vor allem 1 bis 4 C-Atomen und ganz besonders Methyl oder Äthyl, m die Zahl 1 oder 2, vorzugsweise 1, n und p unabhängig voneinander die Zahl 1, 2 oder 3 und vorzugsweise 2 und insbesondere 1 bedeuten.

Dabei können die aromatischen Ringe A, B und D durch $C_{1-3}$-Alkyl und/oder Halogen, besonders Chlor, mono- oder disubstituiert sein. Wenn n und p je die Zahl 1 bedeuten, können die aromatischen Ringe B und D gegebenenfalls durch $C_{1-3}$-Alkyl und/oder Halogen trisubstituiert oder durch $C_{1-3}$-Alkoxy mono- oder disubstituiert sein. Der Ring B' ist gegebenenfalls durch Halogen, besonders Chlor, $C_{1-3}$-Alkyl und/oder $C_{1-3}$-Alkoxy substituiert. Vorzugsweise sind die erwähnten Ringe unsubstituiert. R' und R'' haben vorzugsweise dieselbe Bedeutung.

Des weiteren bevorzug ist die Herstellung von Verbindungen der Formel I, worin Q für die Gruppierung

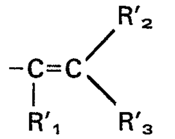

steht, und worin $R_1$ und $R'_1$ bzw. $R_3$ und $R'_3$ je Wasserstoff, $-CN$ oder $C_{1-4}$-Alkyl, $R_2$ und $R'_2$ je $C_{2-4}$-Alkenyl, $-CN$, $-CONR'R''$, $-COOR'''$ oder $-CO-R$ bedeuten, wobei höchstens eines von $R_2$ und $R_3$ und höchstens eines von $R'_2$ und $R'_3$ Wasserstoff darstellen und R, R', R'' und R''' die oben angegebene Bedeutung haben. R stellt dabei bevorzugt $C_{1-6}$-Alkyl oder gegebenenfalls durch Halogen, besonders Chlor, $C_{1-3}$-Alkyl oder $C_{1-3}$-Alkoxy substituiertes Phenyl, insbesondere unsubstituiertes $C_{1-4}$-Alkyl oder gegebenenfalls durch Chlor, Methyl oder Methoxy monosubstituiertes Phenyl dar. R' und R'' sind bevorzugt je Methyl.

R''' stellt vorzugsweise Methyl oder gegebenenfalls durch $C_{1-4}$-Alkoxy oder $C_{3-6}$-Alkoxyalkoxy substituiertes $C_{2-6}$-Alkyl, insbesondere unsubstituiertes $C_{1-4}$-Alkyl dar.

Besonders bevorzugt ist die Herstellung von Verbindungen der Formel I, worin Q für die Gruppierung

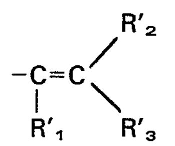

steht, und worin $R_1$ und $R'_1$ je Methyl und insbesondere je Wasserstoff, $R_2$ und $R'_2$ je $-CN$ oder $-COOR'''$, R''' unsubstituiertes Alkyl mit 1 bis 4 C-Atomen und $R_3$ und $R'_3$ je Methyl oder Äthyl und insbesondere je Wasserstoff bedeuten.

Die Verbindungen der Formeln II, III und IV sind bekannt oder können nach an sich bekannten Methoden hergestellt werden. Bezüglich der Herstellung von Verbindungen der Formel IV wird z.B. auf „Houben-Weyl", Bd. 5/1b (1972) verwiesen.

Die Verbindungen der Formeln II und IV werden definitionsgemäss in stöchiometrischer Menge eingesetzt. Bevorzugt verwendet man aber einen Überschuss an Olefin der Formel IV, z.B. bis ca. 1,5 mol Olefin/Säurehalogenid-Gruppe.

X in Formel II stellt vorzugsweise Chlor dar.

Gruppen Y in der Bedeutung von $C_{1-12}$-Alkyl-COO können geradkettig oder verzweigt sein und weisen im Alkyl bevorzugt 1 bis 4 und insbesondere 1 oder 2 C-Atome auf.

Als definitionsgemässe Palladiumverbindungen können – neben Palladiummetall – z.B. Verbindungen der Formel V:

$$M^y\,[PdL_n]^x \qquad (V)$$

eingesetzt werden, worin n eine ganze Zahl von 2 bis 4, x $2^+$ bis $2^-$, $y=-(x)$, M, bei x ungleich 0, ein Gegenion und die L gleiche oder verschiedene phosphorfreie Liganden darstellen, wie z.B. Cl, Br, J, $-CN$, $-NO_3$, $-C_{1-12}$-Alkyl$-COO$, $CH_3COCHCOCH_3$, $NH_3$, 2,2'-Bipyridyl, o-Phenanthrolin, $OS(CH_3)_2$ oder $-NC$-Phenyl. Geeignete Verbindungen der Formel V sind beispielsweise $PdCl_2$, $PdBr_2$, $Pd(CN)_2$, $Pd(NO_3)_2$, $Pd(O_2C-C_{1-12}\text{-alkyl})_2$, besonders $Pd(OOCCH_3)_2$, $Pd(CH_3COCHCHCH_3)_2$, $[Pd(NH_3)_4]Cl_2$, $[PdCl_4]Na_2$, $Pd(OOCCH_3)_2$-(2,2'-bipyridyl), $Pd(OOCCH_3)_2$-(o-phenanthrolin), $PdCl_2[OS(CH_3)_2]_2$ und $PdCl_2$-(NC-phenyl)$_2$.

Ausser den obengenannten Verbindungen können auch Palladiumverbindungen anderer Oxidationsstufen eingesetzt werden, z.B. Bis(dibenzylidenaceton)palladium(O) und Bis(isonitril)palladium(O)verbindungen. Als Beispiele solcher Isonitrile seien genannt:

Bis(cyclohexylisonitril)palladium(O),
Bis(isopropylisonitril)palladium(O),
Bis(tert.-butylisonitril)palladium(O),
Bis-(p-tolylisonitril)palladium(O),
Bis(phenylisonitril)palladium(O) und
Bis-(p-methoxyphenylisonitril)palladium(O).

Davon sind
Bis(dibenzylidenaceton)palladium(O),
Bis(cyclohexylisonitril)palladium(O) und
Bis(isopropylisonitril)palladium(O) bevorzugt.

Bevorzugt verwendet man als Katalysatoren $PdCl_2$, $PdBr_2$, $Pd(OOCCH_3)_2$, $Pd(CH_3COCHCOCH_3)_2$, $Pd(OOCCH_3)_2$-(2,2'-bipyridyl), $PdCl_2$-(NC-phenyl)$_2$, Bis(dibenzylidenaceton)palladium(O) und Bis(cyclohexylisonitril)palladium(O). Ganz besonders bevorzugt sind $PdCl_2$, Palladiumacetat und Bis(dibenzylidenaceton)palladium(O).

Die Katalysatoren werden im allgemeinen in einer Menge von 0,0001 bis 20 Mol.-%, bevorzugt 0,001 bis 3 Mol.-%, bezogen auf die Verbindung der Formel II, eingesetzt.

Als Basen können im erfindungsgemässen Verfahren sowohl anorganische als auch organische Verbindungen, die im Reaktionsmedium ausreichend löslich sind, verwendet. Geeignete Basen sind beispielsweise Verbindungen der Formeln VI bis VIII:

$$(Z)^{q\oplus}(^{\ominus}OOCR_4)_q \qquad (VI)$$

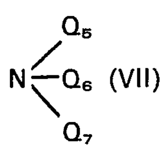 (VII) oder 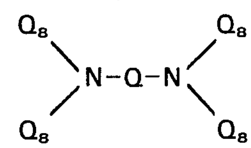 (VIII)

sowie cyclische tertiäre Amine, z.B. N-Methyl- oder N-Äthylpiperidin, 1,2,2,6,6-Pentamethylpiperidin, 4-Oxo-1,2,2,6,6-pentamethylpiperidin, 1,4-Diazabicyclo-[2,2,2]-octan (DABCO), N-Alkylmorpholine und N-Alkylpyrrolidine, wie N-Methyl- und N-Äthylmorpholin, N-Methyl- und N-Äthylpyrrolidin, oder N,N'-Dialkylpiperazine, wie N,N'-Dimethylpiperazin.

In den obigen Formeln bedeuten:
q die Zahl 1 oder 2,
$R_4$ Phenyl oder $C_{1-17}$-Alkyl,
Z ein Alkalimetallkation, ein Erdalkalimetallkation oder

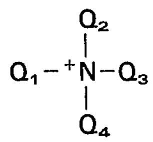

Q geradkettiges oder verzweigtes Alkylen mit 2 bis 6 C-Atomen,

$Q_1$ Wasserstoff, $C_{1-12}$-Alkyl, Cyclopentyl, Cyclohexyl, Benzyl oder Phenyl,

$Q_2$, $Q_3$ und $Q_4$ gleiches oder verschiedenes $C_{1-12}$-Alkyl,

$C_5$ $C_{1-12}$-Alkyl, Cyclopentyl, Cyclohexyl, Phenyl, Benzyl oder durch ein Halogenatom, wie Chlor oder Brom, oder eine Alkyl- oder Alkoxygruppe mit je 1 bis 4 und besonders 1 oder 2 C-Atomen substituiertes Benzyl,

$Q_6$ und $Q_7$ gleiches oder verschiedenes $C_{1-12}$-Alkyl, und

$Q_8$ Methyl oder Äthyl.

Z in der Bedeutung eines Alkalimetallkations ist insbesondere das Natrium- und vor allem das Lithiumkation. Alkylgruppen $R_4$ und $Q_1$ bis $Q_7$ können geradkettig oder verzweigt sein. Stellen $Q_5$ bis $Q_7$ Alkylgruppen dar, so weisen diese mit Vorteil je mindestens 4 C-Atome auf, während Alkylgruppen $Q_1$ bis $Q_4$ bevorzugt je 1 bis 4 C-Atome aufweisen. Beispiele von Verbindungen der Formeln VI bis VIII sind: das Lithiumacetat, -butyrat und -stearat, das Barium- und Calciumacetat, das Kalium-, Calcium- oder Natriumstearat, das Lithium- und Natriumbenzoat sowie die entsprechenden Trimethyl-, Tetramethyl-, Tetraäthyl- und Tetra-n-butylammoniumsalze; Triäthylamin, Tri-n-butylamin, Tri-(2-äthylhexylamin), Tri-n-octylamin und Tri-n-dodecylamin; N,N-Benzyldialkylamine, wie N,N-Benzyldimethylamin, N,N-Benzyldiäthylamin, N,N-(4-Chlorbenzyl)dimethylamin und N,N-(3-Methyl- oder 3-Methoxybenzyl)dimethylamine; N,N,N',N'-Tetramethyl- und N,N,N',N'-Tetraäthyläthylendiamin, N,N,N',N'-Tetramethyl-1,3-diaminopropan und N,N,N',N'-Tetramethyl-1,6-diaminohexan.

Bevorzugt verwendet man als Basen Verbindungen der Formel VII, worin $Q_5$ 4-Chlorbenzyl, 3-Methyl- oder 3-Methoxybenzyl und insbesondere Benzyl und $Q_6$ und $Q_7$ je Alkyl mit 1 bis 4 C-Atomen, vor allem 1 oder 2 C-Atomen darstellen, oder worin $Q_5$, $Q_6$ und $Q_7$ je Alkyl mit 3 bis 12 C-Atomen darstellen. Besonders bevorzugt sind N-Benzyldimethylamin, N-Äthylmorpholin und Tri-n-butylamin.

Die Reaktionstemperaturen für die erfindungsgemässe Umsetzung liegen zweckmässig zwischen 0 und 200° C, vorzugsweise zwischen 90 und 150° C. Sind die Säurehalogenide der Formel II flüssig, so kann die Umsetzung ohne Zusatz eines Lösungsmittels durchgeführt werden. Im allgemeinen wird die Reaktion jedoch in einem gegenüber den Reaktionspartnern inerten organischen Lösungsmittel durchgeführt. Geeignete inerte organische Lösungsmittel sind z.B. gegebenenfalls chlorierte aliphatische oder aromatische Kohlenwasserstoffe, wie n-Pentan, n-Heptan, n-Octan, Benzol, Toluol, Xylole und Chlorbenzol; aromatische, aliphatische und cyclische Äther, wie Anisol, Diäthyläther, Diisopropyläther, Tetrahydrofuran und Dioxan; Nitrile, besonders Benzonitril und Alkylnitrile mit 2 bis 5 C-Atomen, wie Acetonitril, Propionitril und Butyronitril; 3-Methoxypropionitril und 3-Äthoxypropionitril; tertiäre Alkohole mit 4 bis 8 C-Atomen, vor allem tert.-Butanol; aliphatische und cycloaliphatische Ketone, wie Aceton, Diäthylketon, Methylisopropylketon, Cyclopentanon und Cyclohexanon: Ester, wie Ester der Kohlensäure, z.B. Diäthylcarbonat, und Alkyl- und Alkoxyalkyester von aliphatischen Monocarbonsäuren mit insgesamt 2 bis 8 C-Atomen, wie Essigsäuremethyl-, -äthyl-, -n-butyl- und -isobutylester, Buttersäureäthyl- und -n-butylester sowie 1-Acetoxy-2-methoxyäthan. Bevorzugte Lösungsmittel sind Nitrile, Ketone, Ester, cyclische Äther und aromatische Kohlenwasserstoffe der obenerwähnten Art.

Für die Umsetzung in Gegenwart anorganischer Basen eignen sich vor allem polare Lösungsmittel, wie Nitrile, Ketone und Ester. Ganz besonders bevorzugt wird die Umsetzung in Gegenwart einer organischen Base und eines aromatischen Äthers oder Kohlenwasserstoffs, vor allem Anisol, Xylolen oder Toluol, vorgenommen.

Beim erfindungsgemässen Verfahren lässt sich der Reaktionsablauf leicht anhand der CO-Entwicklung verfolgen, beispielsweise mittels eines Blasenzählers. Bei Reaktionsprodukten, die im Reaktionsgemisch nur beschränkt löslich sind, empfiehlt es sich, die Reaktion nach Beendigung der CO-Entwicklung zu unterbrechen und das Reaktionsprodukt direkt aufzuarbeiten.

Die erfindungsgemäss herstellbaren Verbindungen der Formel I können auf an sich bekannt Weise isoliert und gegebenenfalls gereinigt werden. Sie stellen – wie eingangs erwähnt – an sich bekannte optische Aufheller dar, deren Anwendungsgebiete und -formen in der US-PS Nr. 4108887 beschrieben sind.

In einem weiteren Aspekt betrifft die vorliegende Erfindung neue Vinylstilbenverbindungen, welche der Formel IX:

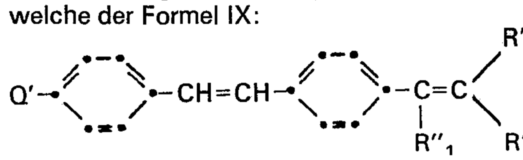 (IX)

entsprechen, worin Q' für –COCl oder

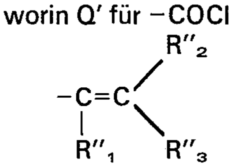

steht, wobei im Falle Q –COCl ist, $R''_1$ und $R''_3$ Wasserstoff und $R''_2$ $-COOCH_3$, $-COOC_2H_5$ oder –CN bedeuten und im Falle Q'

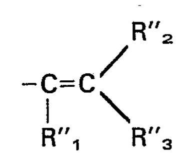

darstellt, R''₁ und R''₃ Wasserstoff und

$R''_2$ $-COO(CH_2)_2CN$, 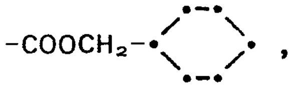 ,

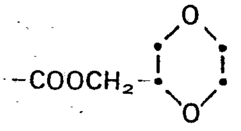 oder 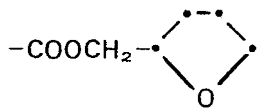

bedeuten.

Die Verbindungen der Formel IX, worin Q' für $-COCl$ steht, sind Zwischenprodukte zur Herstellung bekannter optischer Aufheller, z.B. jener der US-PS Nr. 4108887.

Die Verbindungen der Formel IX, worin Q' für

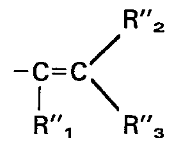

steht, zeigen in gelöstem oder feinverteiltem Zustande eine mehr oder weniger ausgeprägte Fluoreszenz. Sie werden daher erfindungsgemäss zum optischen Aufhellen der verschiedensten synthetischen, halbsynthetischen oder natürlichen organischen Materialien in der Textilindustrie verwendet.

Hierfür seien beispielsweise, ohne dass durch die nachfolgende Übersicht irgendeine Beschränkung hierauf ausgedrückt werden soll, Textilfasern aus folgenden Gruppen von organischen Materialien, soweit eine optische Aufhellung derselben in Betracht kommen, genannt:

I. Synthetische organische hochmolekulare Materialien:

a) Polymerisationsprodukte auf Basis mindestens eine polymerisierbare Kohlenstoff/Kohlenstoff-Doppelbindung enthaltender organischer Verbindungen, d.h. deren Homo- oder Copolymerisate sowie deren Nachbehandlungsprodukte wie beispielsweise Vernetzungs-, Pfropfungs- oder Abbauprodukte, Polymerisatverschnitte oder durch Modifizierung reaktionsfähiger Gruppen erhaltene Produkte, beispielsweise Polymerisate auf Basis von α,β-ungesättigten Carbonsäuren oder Derivaten solcher Carbonsäuren, insbesondere von Acrylverbindungen (wie z.B. Acrylestern, Acrylsäure, Acrylnitril, Acrylamiden und deren Derivaten oder deren Methacrylanaloga), von Olefinkohlenwasserstoffen (wie z.B. Äthylen, Propylen, Styrole oder Diene, ferner sogenannte ABS-Polymerisate) oder Polymerisate auf Basis von Vinyl- und Vinylidenverbindungen (wie z.B. Vinylchlorid, Vinylalkohol, Vinylidenchlorid).

b) Polymerisationsprodukte, die durch Ringöffnung erhältlich sind, z.B. Polyamide vom Polycaprolactamtyp, ferner Polymere, die sowohl über Polyaddition als auch Polykondensation erhältlich sind, wie Polyäther oder Polyacetale.

c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z.B. Äthylenglykolterephthalsäurepolyester) oder ungesättigte (z.B. Maleinsäuredialkoholpolykondensate sowie deren Vernetzungsprodukte mit anpolymerisierbaren Vinylmonomeren), unverzweigte sowie verzweigte (auch auf Basis höherwertiger Alkohole, wie z.B. Alkydharze) Polyester, Polyamide, (z.B. Hexamethylendiaminadipat), Maleinatharze, Melaminharze, deren Vorkondensate und Analoga, Polycarbonate oder Silikone.

d) Polyadditionsprodukte wie Polyurethane (vernetzt und unvernetzt) oder Epoxydharze.

II. Halbsynthetische textile organische Materialien, z.B. auf Basis von Celluloseestern verschiedener Veresterungsgrade (sogenanntes 2-½-Acetat, Triacetat) oder Celluloseäthern, regenerierter Cellulose (Viskose, Kupferammoniakcellulose) oder deren Nachbehandlungsprodukten, ferner Caseinkunststoffen.

III. Natürliche textile organische Materialien animalischen oder vegetabilischen Ursprungs, beispielsweise auf Basis von Cellulose oder Proteinen wie Baumwolle, Wolle, Leinen oder Seide.

Die optisch aufzuhellenden organischen Materialien können verschiedenartigen Verarbeitungszuständen (Rohstoffe, Halbfabrikate oder Fertigfabrikate) angehören. Fasermaterialien können beispielsweise als Stapelfasern, Flocken, Strangware, textile Fäden, Garne, Zwirne, Faservliese, Filze, Watten, Beflockungsgebilde, als textile Gewebe, textile Verbundstoffe oder Gewirke vorliegen.

Den obenstehend definierten Verbindungen kommt besondere Bedeutung für die Behandlung von textilen Geweben zu. Die Behandlung textiler Substrate erfolgt mit Vorteil in wässerigem Medium, worin die betreffenden optischen Aufhellmittel in feinverteilter Form (Suspensionen, sogenannten Mikrodispersionen, gegebenenfalls Lösungen) vorliegen. Gegebenenfalls können bei der Behandlung Dispergiert-, Stabilisier-, Netz- und weitere Hilfsmittel zugegeben werden.

In Abhängigkeit vom verwendeten Aufhellerverbindungstyp kann es sich als vorteilhaft erweisen, vorzugsweise in neutralem, in alkalischem oder in saurem Bade zu arbeiten. Die Behandlung wird üblicherweise bei Temperaturen von etwa 20 bis 140° C, beispielsweise bei Siedetemperatur des Bades oder in deren Nähe (etwa 90° C), durchgeführt. Für die erfindungsgemässe Veredlung textiler Substrate kommen auch Lösungen oder Emulsionen in organischen Lösungsmitteln in Betrach, wie dies in der Färbereipraxis in der sogenannten Lösungsmittelfärberei (Foulardthermofixierapplikation, Ausziehfärbeverfahren in Färbemaschinen) praktiziert wird.

Die neuen, gemäss vorliegender Erfindung verwendbaren optischen Aufhellmittel können beispielsweise auch in folgenden Anwendungsformen eingesetzt werden:

a) In Mischungen mit Farbstoffen (Nuancierung) oder Pigmenten (Farb- oder insbesondere z.B. Weisspigmenten) oder als Zusatz zu Färbebädern, Druck-, Ätz- oder Reservepasten. Ferner auch zur Nachbehandlung von Färbungen, Drucken oder Ätzdrucken.

b) In Mischungen mit sogenannten Carriern, Netzmitteln, Weichmachern, Quellmitteln, Antioxydantien, Lichtschutzmitteln, Hitzestabilisatoren, chemischen Bleichmitteln (Chloritbleiche, Bleichbäderzusätze).
c) In Mischung mit Vernetzern, Appreturmitteln (z.B. Stärke oder synthetischen Appreturen) sowie in Kombination mit den verschiedensten Textilveredlungsverfahren, insbesondere Kunstharzausrüstungen (z.B. Knitterfestausrüstungen wie *wash-and-wear, permanent-press, no-iron*), ferner Flammfest-, Weichgriff-, Schmutzablöse *(antisoiling)*- oder Antistatischausrüstungen oder antimikrobiellen Ausrüstungen.
d) Einarbeiten der optischen Aufhellmittel in polymere Trägermaterialien (Polymerisations-, Polykondensations- oder Polyadditionsprodukte) in gelöster oder dispergierter Form für Anwendung z.B. in Beschichtungs-, Imprägnier- oder Bindemitteln (Lösungen, Dispersionen, Emulsionen) für Textilien, Faservliese usw.
e) Als Zusätze zu sogenannten *master batches.*
f) Als Zusätze zu verschiedenen Seifen und Waschmitteln.
g) In Kombination mit anderen, optisch aufhellend wirkenden Substanzen.

Wird das Aufhellverfahren mit Textilbehandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

In gewissen Fällen werden die Aufheller durch eine Nachbehandlung zur vollen Wirkung gebracht. Diese kann beispielsweise eine chemische (z.B. Säurebehandlung), eine thermische (z.B. Hitze) oder eine kombinierte chemisch-thermische Behandlung darstellen. So verfährt man beispielsweise beider optischen Aufhellung einer Reihe von Fasersubstraten, z.B. von Polyesterfasern, mit den erfindungsgemässen Aufhellern zweckmässig in der Weise, dass man diese Fasern mit den wässerigen Dispersionen (gegebenenfalls auch Lösungen) der Aufhellmittel bei Temperaturen unter 75° C, z.B. bei Raumtemperatur, imprägniert und einer trockenen Wärmebehandlung bei Temperaturen über 100° C unterwirft, wobei es sich im allgemeinen empfiehlt, das Fasermaterial vorher noch bei mässig erhöhter Temperatur, z.B. bei mindestens 60 bis etwa 130° C zu trocknen. Die Wärmebehandlung in trockenem Zustande erfolgt dann vorteilhaft bei Temperaturen zwischen 120 und 225° C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einen einzigen Arbeitsgang zusammengelegt werden.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,0001 Gew.-%, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,0005 und 0,5 Gewichtsprozent von Interesse.

Die neuen optischen Aufhellmittel eignen sich auch besonders als Zusätze für Waschbäder oder zu Waschmitteln, wobei sie in verschiedener Weise zugesetzt werden können. Zu Waschbädern werden sie zweckmässig in Form ihrer Lösungen in Wasser oder organischen Lösungsmitteln oder auch in feiner Verteilung als wässerige Dispersionen zugegeben. Zu Waschmitteln werden sie vorteilhaft in irgendeiner Phase des Herstellungsprozesses der Waschmittel, z.B. dem sogenannten *slurry* vor dem Zerstäuben des Waschpulvers oder bei der Vorbereitung flüssiger Waschmittelkombinationen zugesetzt. Die Zugabe kann sowohl in Form einer Lösung oder Dispersion in Wasser oder andern Lösungsmitteln als auch ohne Hilfsmittel als trockenes Aufhellerpulver erfolgen. Man kann die Aufhellmittel beispielsweise mit den waschaktiven Substanzen vermischen, verkneten oder vermahlen und so dem fertigen Waschpulver zumischen. Sie können jedoch auch gelöst oder vordispergiert auf das fertige Waschmittel aufgesprüht werden.

Als Waschmittel kommen die bekannten Mischungen von Waschaktivsubstanzen wie beispielsweise Seife in Form von Schnitzeln und Pulver, Synthetika, lösliche Salze von Sulfonsäurehalbestern höherer Fettalkohole, höher und/oder mehrfach alkylsubstituierter Arylsulfonsäuren, Sulfocarbonsäureester mittlerer bis höherer Alkohole, Fettsäureacylaminoalkyl- oder -aminoarylglycerinsulfonate, Phosphorsäureester von Fettalkoholen usw., in Frage. Als Aufbaustoffe, sogenannte *builders,* kommen z.B. Alkalipoly- und -polymetaphosphate, Alkalipyrophosphate, Alkalisalze der Carboxymethylcellulose und andere *soilredeposition*-Inhibitoren, ferner Alkalisilikate, Alkalicarbonate, Alkaliborate, Alkaliperborate, Nitrilotriessigsäure, Äthylendiaminotetraessigsäure, Schaumstabilisatoren wie Alkanolamide höherer Fettsäuren, in Betracht. Ferner können in den Waschmitteln beispielsweise enthalten sein: Antistatische Mittel, rückfettende Hautschutzmittel wie Lanolin, Enzyme, Antimikrobika, Parfüme und Farbstoffe.

Die neuen optischen Aufheller haben den besonderen Vorteil, dass sie auch bei Gegenwart von Aktivchlorspendern, wie z.B. Hypochlorit, wirksam sind und ohne wesentliche Einbusse der Effekte in Waschbädern mit nichtionogenen Waschmitteln, z.B. Alkylphenolpolyglykoläthern, verwendet werden können.

Waschflotten, die die angegebenen Mengen der erfindungsgemäss anwendbaren optischen Aufheller enthalten, verleihen beim Waschen von Textilien aus Cellulosefasern, Polyamidfasern, hochveredelten Cellulosefasern, Polyesterfasern, Wolle usw., einen brillanten Aspekt am Tageslicht.

Die Waschbehandlung wird beispielsweise wie folgt durchgeführt:

Die angegebenen Textilien werden während 1 bis 30 min bei 20 bis 100° C in einem Waschbad behandelt, das 1 bis 10 g/kg eines aufgebauten, zusammengesetzten Waschmittels und 0,05 bis 1%, bezogen auf das Waschmittelgewicht, der beanspruchten Aufhellmittel enthält. Das Flottenverhältnis kann 1 :2 bis 1 :50 betragen. Nach dem Waschen wird wie üblich gespült und getrocknet. Das Waschbad kann als Bleichzusatz 0,2 g/l Aktivchlor (z.B. als Hypochlorit) oder 0,1 bis 2 g/l Natriumperborat enthalten.

In den nachfolgenden Beispielen sind Prozente immer Gewichtsprozente. Schmelz- und Siedepunkte sind, sofern nicht anders vermerkt, unkorrigiert.

*Beispiel 1:*

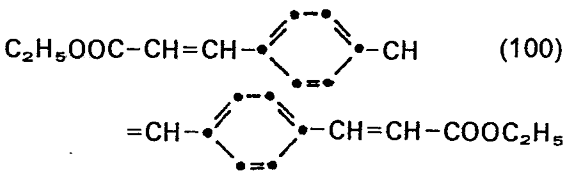

(100)

6,1 g (0,02 mol) Stilben-4,4'-dicarbonsäurechlorid (hergestellt gemäss Fosdick, Urbach, „J. Am. Chem. Soc.", *69*, 503 [1947], Hager, Shonle, „J. Am. Chem. Soc.", *68*, 2167 [1946] und „J. Chem. Soc.", 1024 [1948]) werden zusammen mit 5,2 g (0,05 mol) Acrylsäureäthylester, 5,06 g (0,05 mol) Triäthylamin, 20 ml Toluol und 0,089 g (0,0004 mol) Palladiumacetat in einen 100-ml-Kolben eingefüllt und unter Rühren auf 100° C (Badtemperatur) erwärmt. Es ist eine leichte Gasentwicklung zu beobachten. Nach einer Rührzeit von 6 h wird das Reaktionsprodukt mit Toluol extrahiert, und die vereinigten Extrakte werden zur Trockne eingeengt und aus Benzol umkristallisiert. Man erhält 3,4 g Stilben-4,4'-bisacrylsäureäthylester; Ausbeute 45% d.Th.; Fp. 245-248° C (grüngelbe Blättchen).

*Analyse* (Molgewicht 376,45):

| | | | |
|---|---|---|---|
| Berechnet: | C 76,57 | H 6,43 | O 17,00% |
| Gefunden: | C 76,82 | H 6,24 | O 17,03% |

*Beispiel 2:*

Es wird wie in Beispiel 1 beschrieben vorgegangen, jedoch unter Verwendung von 9,25 g (0,05 mol) Tri-n-butylamin anstelle von 5,06 g (0,05 mol) Triäthylamin. Nach der Aufarbeitung wie in Beispiel 1 beschrieben erhält man den Stilben-4,4'-bisacrylsäureäthylester in einer Ausbeute von 56% d.Th.

Verfährt man wie oben beschrieben, jedoch unter Verwendung der entsprechenden Olefinen, so erhält man die in der nachfolgenden Tabelle aufgeführten Verbindungen der Formel:

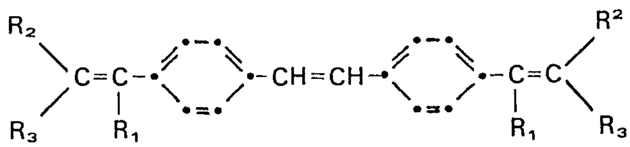

| Verbindung Nr. | $R_1$ | $R_2$ | $R_3$ | Schmelzpunkt (°C) |
|---|---|---|---|---|
| 101 | H | 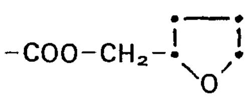 | H | 152-154 |
| 102 | H | $-COO(CH_2)_2-O(CH_2)_3-OCH_3$ | H | 122-124 |
| 103 | H | 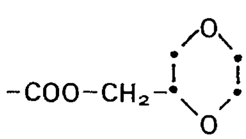 | H | 180-181 |
| 104 | H | 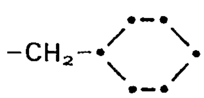 | H | 169-170 |
| 105 | CN | $-(CH_2)_2-CN$ | H | 230-232 |
| 106 | H | CN | CN | >300 |
| 107 | H | $-CO-C_2H_5$ | H | 244-246 |
| 108 | H | $-CON(C_2H_5)_2$ | H | 210-211 |

| Verbindung Nr. | $R_1$ | $R_2$ | $R_3$ | Schmelzpunkt (°C) |
|---|---|---|---|---|
| 109 | $-COOCH_3$ | $-CH_2COOCH_3$ | H | 157-158 |
| 110 | H | $-COOCH_3$ | H | 269-270 |
| 111 | H | $-COO(CH_2)_2CN$ | H | 213-215 |

*Beispiel 3:*

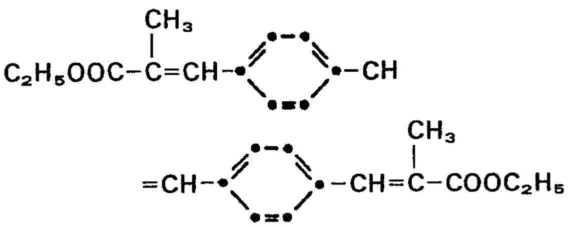

Es wird wie in Beispiel 1 beschrieben vorgegangen, jedoch unter Verwendung von 8,7 g (0,05 mol) Methacrylsäureäthylester anstelle von Acrylsäureäthylester. Nach der Aufarbeitung erhält man den Stilben-4,4'-bismethacrylsäureäthylester in Form von grünlich gelben Blättchen; Fp. 123-125° C.

*Analyse* (Molgewicht 404):

| | | | |
|---|---|---|---|
| Berechnet: | C 77,17 | H 7,02 | O 15,81% |
| Gefunden: | C 77,27 | H 7,08 | O 15,65% |

*Beispiel 4:*

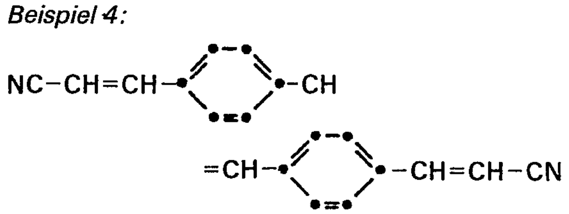

Es wird wie in Beispiel 1 beschrieben vorgegangen, jedoch unter Verwendung von 2,65 g (0,05 mol) Acrylnitril anstelle von Acrylsäureäthylester. Nach der Aufarbeitung erhält man das Stilben-4,4'-bisacrylnitril in Form von grünlich gelben Kristallen; Fp. 219-221° C.

*Analyse* (Molgewicht 282):

| | | | |
|---|---|---|---|
| Berechnet: | C 85,08 | H 5,00 | N 9,92% |
| Gefunden: | C 84,75 | H 5,07 | N 9,67% |

*Beispiel 5:*

Es wird wie in Beispiel 1 beschrieben vorgegangen, jedoch unter Verwendung von 0,0112 g (0,00005 mol) Palladiumacetat und 6,76 g (0,05 mol) N-Benzyldimethylamin. Man erhält 1,54 g der Verbindung 100.

*Beispiel 6:*

Es wird wie in Beispiel 1 beschrieben vorgegangen, jedoch unter Verwendung von 6,76 g (0,05 mol) N-Benzyldimethylamin. Nach der Aufarbeitung erhält man 2,9 g der Verbindung 100 entsprechend einer Ausbeute von 76% d. Th.

*Beispiel 7:*

Es wird wie in Beispiel 1 beschrieben vorgegangen, jedoch unter Verwendung von 0,0561 g (0,000025 mol) Palladiumacetat, 50 ml Xylol, 7,63 g (0,025 mol) Stilben-4,4'-dicarbonsäurechlorid, 2,60 g (0,025 mol) Acrylsäureäthylester und 3,38 g (0,025 mol) N-Benzyldimethylamin. Man erhält 0,17 g der Verbindung der Formel 700:

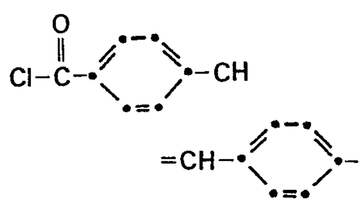

(700)

in Form von schwach gelben Kristallen; Fp. 143° C.

*Beispiel 8:*

Es wird wie in Beispiel 7 beschrieben vorgegangen, jedoch unter Verwendung von 1,33 g (0,025 mol) Acrylnitril anstelle von 2,60 g (0,025 mol) Acrylsäureäthylester. Nach der Aufarbeitung erhält man die Verbindung der Formel:

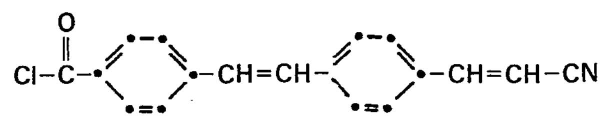

in Form von gelben Kristallen.

*Beispiel 9:*

Es wird wie in Beispiel 1 beschrieben vorgegangen, jedoch unter Verwendung von 0,0059 g (0,0000264 mol) Palladiumacetat, 0,45 g (0,00132 mol) der Verbindung der Formel 700, 0,172 g (0,00165 mol) Styrol, 0,18 g (0,00132 mol) N-Benzyldimethylamin in 3 ml p-Xylol als Lösungsmittel. Nach der Aufarbeitung erhält man 0,09 g der Verbindung der Formel:

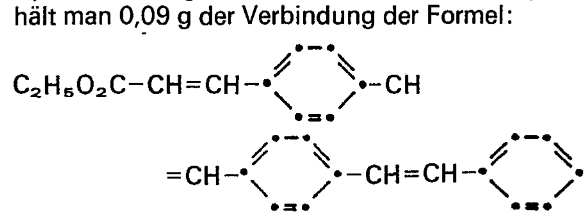

in Form von geblichen Kristallen.

*Beispiel 10:*

Man foulardiert bei Raumtemperatur ein Polyestergewebe (Terylen Typ 540) mit einer Flotte, welche 1 g/l der Verbindung der Formel 101, 103,

104 oder 111 und 1 ml des Kondensationsproduktes von 8-9 mol Äthylenoxid mit 1 mol p-tert.-Octylphenol enthält. Der Abquetscheffekt beträgt 80%. Anschliessend wird 10 min bei 80° C getrocknet und dann 30 s bei 200° C thermofixiert. Das so behandelte Gewebe weist einen hohen Aufhelleffekt auf.

*Beispiel 11:*

Ein Polyester/Baumwoll-Mischgewebe wird auf einem Färbeapparat bei einem Flottenverhältnis von 1:20 mit einem wässerigen Bad behandelt, welches 0,1%, bezogen auf das Warengewicht, der Verbindung der Formel 101, 103, 104 oder 111 und 1 g/l des Kondensationsproduktes von 35 mol Äthylenoxid mit 1 mol Stearylalkohol enthält. Man erwärmt nun das Bad innerhalb 30 min von 40 auf 97° C, behält es während 30 min auf dieser Temperatur und kühlt es dann innerhalb 15 min auf 15° C ab. Das Gewebe wird anschliessend in fliessendem, deionisiertem Wasser gespült und bei 70° C getrocknet. Das so behandelte Polyester/Baumwoll-Mischgewebe zeichnet sich durch einen hohen Aufhelleffekt aus.

**Patentansprüche**

1. Verfahren zur Herstellung von Vinylstilbenverbindungen der Formel (IX):

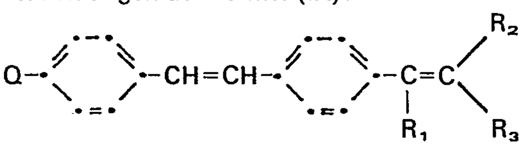 (IX)

worin Q für die Gruppierung $-COX$ oder

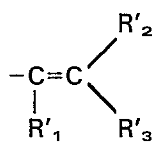 steht, und worin $R_1$ und $R'_1$ unabhängig voneinander Wasserstoff, Cyano, Alkyl oder eine nicht chromophore veresterte Carboxylgruppe, $R_2$ und $R'_2$ unabhängig voneinander Wasserstoff, gegebenenfalls nicht chromophor substituiertes Alkyl oder Alkenyl oder einen nicht chromophoren Substituenten zweiter Ordnung, $R_3$ und $R'_3$ unabhängig voneinander Wasserstoff, Cyano oder gegebenenfalls nicht chromophor substituiertes Alkyl oder Alkenyl, und X Chlor, Brom oder Jod bedeuten oder $R_1$ zusammen mit $R_3$ bzw. $R'_1$ zusammen mit $R'_3$ eine Alkylenbrücke darstellen, wobei höchstens eines der Symbole $R_2$ und $R_3$ und höchstens eines der Symbole $R'_2$ und $R'_3$ Wasserstoff sind und wobei, wenn Q für $-COX$ steht, $R_1$ und $R_3$ Wasserstoff und $R_2$ Carbonsäure-$C_{1-4}$-alkylester oder Cyano bedeuten, dadurch gekennzeichnet, dass man 1 mol-Eq einer Verbindung der Formel (II):

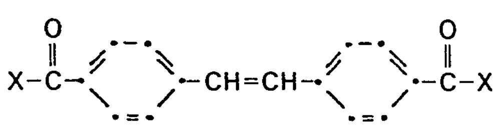 (II)

worin X die oben angegebene Bedeutung hat, in Gegenwart einer Base und unter Zusatz von Palladiummetall oder von Palladiumverbindungen, die unter den Reaktionsbedingungen phosphorfreie labile Palladium(O)verbindungen bilden, als Katalysator mit 1 mol-Eq einer Verbindung der Formel (III) oder mit je 1 mol-Eq der Verbindungen der Formeln (III) und (IV):

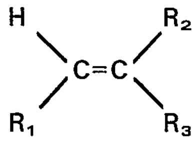 (III) 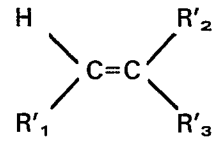 (IV)

worin $R_1$, $R_2$ und $R_3$ bzw. $R'_1$, $R'_2$ und $R'_3$ die oben angegebene Bedeutung haben, umsetzt.

2. Verfahren nach Anspruch 1, dadurch gekennzeichnet, dass man eine Verbindung der Formel (II) verwendet, worin X Chlor darstellt.

3. Verfahren nach Anspruch 1, dadurch gekennzeichnet, dass man eine Verbindung der Formel (I) herstellt, worin Q für die Gruppierung:

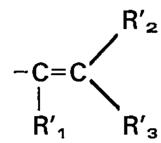

steht und worin $R_1$ und $R'_1$ unabhängig voneinander Wasserstoff, Cyano, $C_{1-6}$-Alkyl oder $-COOR'''$, $R_2$ und $R'_2$ unabhängig voneinander Wasserstoff oder gegebenenfalls durch $C_{1-4}$-Alkoxy, oder $C_{2-5}$-Alkoxycarbonyl substituiertes $C_{1-6}$-Alkyl, $C_{2-4}$-Alkenyl, $-CN$, $-CF_3$ oder eine Gruppe:

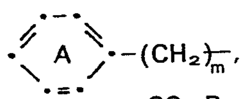

$-CO-R$, $-CO-NR'R''$ oder $-COOR'''$

$R_3$ und $R'_3$ unabhängig voneinander Wasserstoff, Cyano oder gegebenenfalls durch $C_{1-4}$-Alkoxy oder $C_{2-5}$-Alkoxycarbonyl substituiertes $C_{1-6}$-Alkyl, oder $R_1$ und $R_3$ bzw. $R'_1$ und $R'_3$ zusammen $-(CH_2)_3$ oder $-(CH_2)_4$ darstellen, wobei höchstens eines von $R_2$ und $R_3$ und höchstens eines von $R'_2$ und $R'_3$ Wasserstoff sind, R Methyl, gegebenenfalls durch $C_{1-4}$-Alkoxy, $C_{4-6}$-Alkoxyalkoxy substituiertes $C_{2-6}$-Alkyl, einen Rest der Formel:

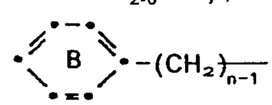

oder Naphthyl, R' Wasserstoff, Methyl, gegebenenfalls durch $C_{1-4}$-Alkoxy, $C_{4-6}$-Alkoxyalkoxy oder Di-($C_{1-4}$-alkyl)amino substituiertes $C_{2-6}$-Alkyl, gegebenenfalls methyl- oder äthylsubstituiertes Cyclohexyl oder einen Rest der Formel:

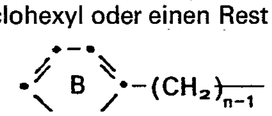

R'' Wasserstoff, Methyl oder gegebenenfalls durch $C_{1-4}$-Alkoxy oder $C_{4-6}$-Alkoxyalkoxy substituiertes $C_{2-6}$-Alkyl, R''' Methyl, gegebenenfalls durch $C_{1-4}$-Alkoxy oder $C_{3-6}$-Alkoxyalkoxy substi-

tuiertes $C_{2-6}$-Alkyl, gegebenenfalls methyl- und/oder äthylsubstituiertes Cyclohexyl oder einen Rest der Formel:

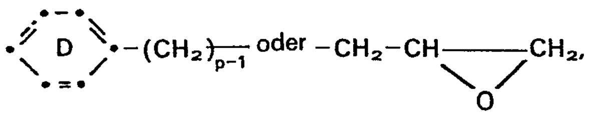

m die Zahl 1 oder 2 und n und p unabhängig voneinander die Zahl 1, 2 oder 3 bedeuten, wobei die Ringe A, B und C durch $C_{1-3}$-Alkyl und/oder Halogen mono- oder disubstituiert und die Ringe B und D, wenn n und p je die Zahl 1 bedeuten, durch $C_{1-3}$-Alkyl und/oder Halogen trisubstituiert oder durch $C_{1-3}$-Alkoxy mono- oder disubstituiert sein können.

4. Verfahren nach Anspruch 3, dadurch gekennzeichnet, dass man eine Verbindung der Formel (I) herstellt, worin Q für die Gruppierung:

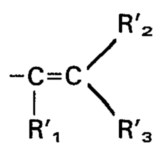

steht und worin $R_1$ und $R'_1$ unabhängig voneinander Wasserstoff, $-CN$ oder $C_{1-4}$-Alkyl, $R_2$ und $R'_2$ unabhängig voneinander $C_{2-4}$-Alkenyl, $-CN$, $-CONR'R''$, $-COOR'''$ oder $-CO-R$ und $R_3$ und $R'_3$ unabhängig voneinander Wasserstoff, $-CN$ oder $C_{1-4}$-Alkyl bedeuten, wobei höchstens eines von $R_2$ und $R_3$ und höchstens eines von $R'_2$ und $R'_3$ Wasserstoff sind, R Methyl, gegebenenfalls durch $C_{1-4}$-Alkoxy oder $C_{4-6}$-Alkoxyalkoxy substituiertes $C_{2-6}$-Alkyl, Naphthyl oder gegebenenfalls durch Halogen, $C_{1-3}$-Alkyl und/oder $C_{1-3}$-Alkoxy substituiertes Phenyl, R' und R'' je Methyl, R''' Methyl oder gegebenenfalls durch $C_{1-4}$-Alkoxy oder $C_{3-6}$-Alkoxyalkoxy substituiertes $C_{2-6}$-Alkyl, m die Zahl 1, n und p unabhängig voneinander die Zahl 2 und insbesondere die Zahl 1 darstellen.

5. Verfahren nach Anspruch 3, dadurch gekennzeichnet, dass man eine Verbindung der Formel (I) herstellt, worin Q für die Gruppierung:

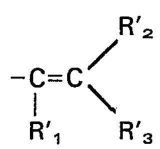

steht und worin $R_1$ und $R'_1$ bzw. $R_3$ und $R'_3$ je Wasserstoff, $-CN$ oder $C_{1-4}$-Alkyl, $R_2$ und $R'_2$ je $C_{2-4}$-Alkenyl, $-CN$, $-CONR'R''$, $-COOR'''$ oder $-CO-R$ bedeuten, wobei höchstens eines von $R_2$ und $R_3$ und höchstens eines von $R'_2$ und $R'_3$ Wasserstoff darstellen, R $C_{1-6}$-Alkyl oder gegebenenfalls durch Halogen, $C_{1-3}$-Alkyl oder $C_{1-3}$-Alkoxy substituiertes Phenyl, insbesondere unsubstituiertes $C_{1-4}$-Alkyl oder gegebenenfalls durch Chlor, Methyl oder Methoxy monosubstituiertes Phenyl, R' und R'' je Methyl und R''' Methyl oder gegebenenfalls durch $C_{1-4}$-Alkoxy oder $C_{3-6}$-Alkoxyalkoxy substituiertes $C_{2-6}$-Alkyl, insbesondere unsubstituiertes $C_{1-4}$-Alkyl bedeuten.

6. Verfahren nach Anspruch 3, dadurch gekennzeichnet, dass man eine Verbindung der Formel (I) herstellt, worin Q für die Gruppierung:

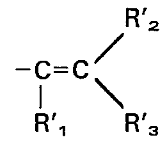

worin $R_1$ und $R'_1$ je Methyl und insbesondere je Wasserstoff, $R_2$ und $R'_2$ je $-CN$ oder $-COOR'''$, R''' unsubstituiertes Alkyl mit 1 bis 4 C-Atomen und $R_3$ und $R'_3$ je Methyl oder Äthyl und insbesondere Wasserstoff bedeuten.

7. Verfahren nach Anspruch 1, dadurch gekennzeichnet, dass man eine Verbindung der Formel (I) herstellt, worin Q für $-COCl$ steht, und $R_1$ und $R_3$ Wasserstoff und $R_2$ Carbonsäure-$C_{1-4}$-alkylester oder Cyano bedeuten.

8. Verfahren nach Anspruch 1, dadurch gekennzeichnet, dass man als Verbindung der Formel (III) $PdCl_2$, Palladiumacetylacetonat und insbesondere Palladiumacetat verwendet.

9. Verfahren nach Anspruch 1, dadurch gekennzeichnet, dass man die Umsetzung bei einer Temperatur zwichen 0 und 200° C, insbesondere zwischen 90 und 150° C und in Gegenwart eines gegenüber den Reaktionspartnern inerten organischen Lösungsmittels vornimmt.

10. Verfahren nach Anspruch 9, dadurch gekennzeichnet, dass man als Lösungsmittel Anisol, Xylol oder Toluol verwendet.

11. Verfahren nach Anspruch 1, dadurch gekennzeichnet, dass man als Base ein Trialkylamin mit je 4 bis 12 C-Atomen in den Alkylresten verwendet.

12. Verfahren nach Anspruch 1, dadurch gekennzeichnet, dass man den Katalysator in einer Menge von 0,0001 bis 20 Mol.-%, bezogen auf die Verbindung der Formel (II), verwendet.

13. Vinylstilbenverbindungen der Formel:

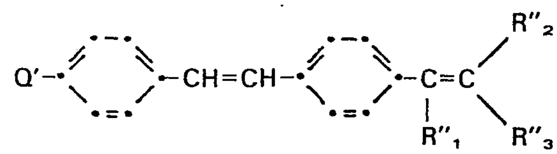

worin Q' für $-COCl$ oder

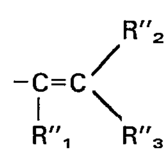

steht, wobei im Falle Q' $-COCl$ ist, $R''_1$ und $R''_3$ Wasserstoff und $R''_2$ $-COOCH_3$, $-COOC_2H_5$ oder $-CN$ bedeuten und im Falle Q'

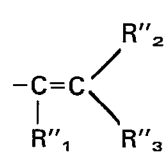

darstellt, $R''_1$ und $R''_3$ Wasserstoff und $R''_2$

$-COO(CH_2)_2CN$, $-COOCH_2-$ 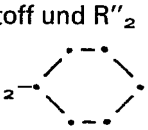 ,

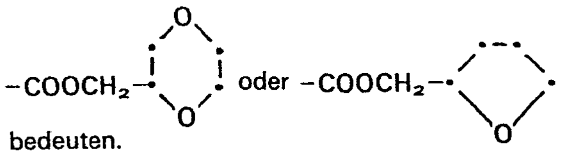

bedeuten.

14. Verfahren zum optischen Aufhellen von synthetischen, halbsynthetischen und natürlichen hochmolekularen organischen Materialien, dadurch gekennzeichnet, dass man den optisch aufzuhellenden Materialien Vinylstilbenverbindungen der in Anspruch 13 definierten Formel, worin Q' für

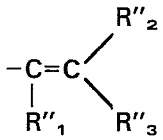

steht, einverleibt oder auf die Oberfläche besagter Materialien aufbringt.

15. Verfahren nach Anspruch 14, dadurch gekennzeichnet, dass man 0,001 bis 2%, vorzugsweise 0,01 bis 0,5% des Aufhellers, bezogen auf das Gewicht des aufzuhellenden Materials, verwendet.

16. Verfahren nach Anspruch 15 zum optischen Aufhellen von Polyestern.

17. Verfahren nach Anspruch 16, dadurch gekennzeichnet, dass man Gewebe aus Polyesterfasern mit Hilfe des Ausziehverfahrens oder des Foulardthermoverfahrens mit dem jeweiligen Aufheller behandelt.

## Claims

1. A process for the production of a vinylstilbene compound of the formula (IX):

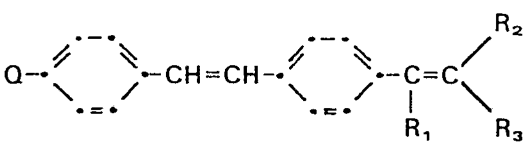 (IX)

wherein Q is the grouping $-COX$ or

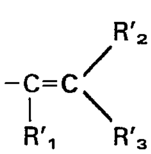 and wherein each of $R_1$ and $R'_1$ independently is hydrogen, cyano, alkyl or a non-chromophoric esterified carboxyl group, each of $R_2$ and $R'_2$ independently is hydrogen, alkyl or alkenyl each unsubstituted or substituted by non-chromophoric substituents, or is a non-chromophoric second order substituent, each of $R_3$ and $R'_3$ independently is hydrogen, cyano, or alkyl or alkenyl each unsubstituted or substituted by non-chromophoric substituents, and X is chlorine, bromine or iodine; or $R_1$ together with $R_3$ or $R'_1$ together with $R'_3$ are an alkylene bridge, with the proviso that not more than one of $R_2$ and $R_3$ and not more than one of $R'_2$ and $R'_3$ are hydrogen, and that, if Q is $-COX$, $R_1$ and $R_3$ are hydrogen and $R_2$ is carboxylic acid $C_1$-$C_4$-alkyl ester or cyano, which process comprises reacting 1 mol-Eq of a compound of the formula (II):

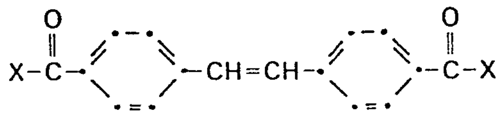 (II)

wherein X is as defined above, in the presence of a base and with the addition of palladium metal or of palladium compounds which, under the reaction conditions, form labile palladium(O) compounds which do not contain phosphorus, with 1 mol-Eq of a compound of the formula (III) or with 1 mol-Eq of each of the compounds of the formulae (III) and (IV):

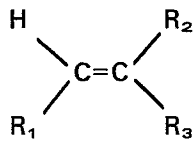 (III) 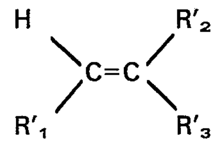 (IV)

wherein $R_1$, $R_2$ and $R_3$ and $R'_1$, $R'_2$ and $R'_3$ are as defined above.

2. A process according to Claim 1 which comprises the use of a compound of the formula (II), wherein X is chlorine.

3. A process according to Claim 1 for the production of a compound of the formula (I), wherein Q is the grouping:

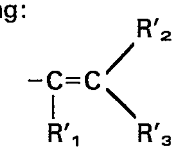

and wherein each of $R_1$ and $R'_1$ independently is hydrogen, cyano, $C_1$-$C_6$-alkyl or $-COOR'''$, each of $R_2$ and $R'_2$ independently is hydrogen or $C_1$-$C_6$-alkyl unsubstituted or substituted by $C_1$-$C_4$-alkoxy or $C_2$-$C_5$-alkoxycarbonyl or is $C_2$-$C_4$-alkenyl, $-CN$, $-CF_3$ or a group:

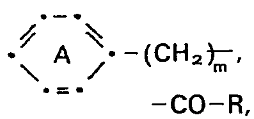

$-CO-R$, $-CO-NR'R''$ or $-COOR'''$

each of $R_3$ and $R'_3$ independently is hydrogen, cyano or $C_1$-$C_6$-alkyl unsubstituted or substituted by $C_1$-$C_4$-alkoxy or $C_2$-$C_5$-alkoxycarbonyl, or $R_1$ and $R_3$ and $R'_1$ and $R'_3$ together are $-(CH_2)_3$ or $-(CH_2)_4$, with the proviso that not more than one of $R_2$ and $R_3$ and not more than one of $R'_2$ and $R'_3$ are hydrogen, R is methyl, $C_2$-$C_6$-alkyl unsubstituted or substituted by $C_1$-$C_4$-alkoxy or $C_4$-$C_6$-alkoxyalkoxy, a radical of the formula:

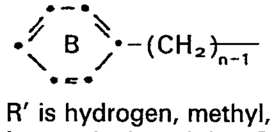

or naphthyl; R' is hydrogen, methyl, $C_2$-$C_6$-alkyl unsubstituted or substituted by $C_1$-$C_4$-alkoxy, $C_4$-$C_6$-alkoxyalkoxy or di-($C_1$-$C_4$-alkyl)amino, unsubstituted or methyl- or ethyl-substituted cyclohexyl or a radical of the formula:

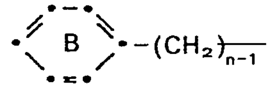

R'' is hydrogen, methyl or $C_2$-$C_6$-alkyl unsubstituted or substituted by $C_1$-$C_4$-alkoxy or $C_4$-$C_6$-alkoxyalkoxy; R''' is methyl, $C_2$-$C_6$-alkyl unsubstituted or substituted by $C_1$-$C_4$-alkoxy or $C_3$-$C_6$-alkoxyalkoxy, unsubstituted or methyl- and/or ethyl-substituted cyclohexyl or a radical of the formula:

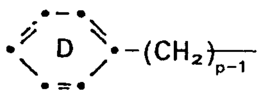 or 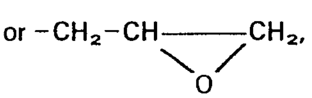

m is 1 or 2, each of n and p independently is 1, 2 or 3, whilst the rings A, B, C and D can be mono- or disubstituted by $C_1$-$C_3$-alkyl and/or halogen, and, if each of n and p is 1, the aromatic rings B and D can be trisubstituted by $C_1$-$C_3$-alkyl and/or halogen, or mono- or disubstituted by $C_1$-$C_3$-alkoxy.

4. A process according to Claim 3 for the production of a compound of the formula (I), wherein Q is the grouping:

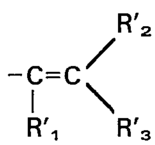

and wherein each of $R_1$ and $R'_1$ independently is hydrogen, $-CN$ or $C_1$-$C_4$-alkyl; each of $R_2$ and $R'_2$ independently is $C_2$-$C_4$-alkenyl, $-CN$, $-CONR'R''$, $-COOR'''$ or $-CO-R$; and each of $R_3$ and $R'_3$ independently is hydrogen, $-CN$ or $C_1$-$C_4$-alkyl, whilst not more than one of $R_2$ and $R_3$ and not more than one of $R'_2$ and $R'_3$ are hydrogen; R is methyl, $C_2$-$C_6$-alkyl unsubstituted or substituted by $C_1$-$C_4$-alkoxy or $C_4$-$C_6$-alkoxyalkoxy, or is naphthyl, or phenyl unsubstituted or substituted by halogen, $C_1$-$C_3$-alkyl and/or $C_1$-$C_3$-alkoxy; each of R' and R'' is methyl; R''' is methyl or $C_2$-$C_6$-alkyl unsubstituted or substituted by $C_1$-$C_4$-alkoxy or $C_3$-$C_6$-alkoxyalkoxy; m is 1, each of n and p independently is 2 and, in particular, 1.

5. A process according to Claim 3 for the production of a compound of the formula (I), wherein Q is the grouping:

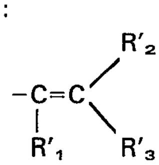

and wherein each of $R_1$ and $R'_1$ and $R_3$ and $R'_3$ is hydrogen, $-CN$ or $C_1$-$C_4$-alkyl, each of $R_2$ and $R'_2$ is $C_2$-$C_4$-alkenyl, $-CN$, $-CONR'R''$, $-COOR'''$ or $-CO-R$, whilst not more than one of $R_2$ and $R_3$ and not more than one of $R'_2$ and $R'_3$ are hydrogen; R is $C_1$-$C_6$-alkyl or phenyl unsubstituted or substituted by halogen, $C_1$-$C_3$-alkyl or $C_1$-$C_3$-alkoxy, in particular unsubstituted $C_1$-$C_4$-alkyl or phenyl unsubstituted or monosubstituted by chlorine, methyl or methoxy; each of R' and R'' is methyl and R''' is methyl or $C_2$-$C_6$-alkyl unsubstituted or substituted by $C_1$-$C_4$-alkoxy or $C_3$-$C_6$-alkoxyalkoxy, especially unsubstituted $C_1$-$C_4$-alkyl.

6. A process according to Claim 3 for the production of a compound of the formula (I), wherein Q is the grouping:

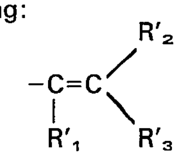

and wherein each of $R_1$ and $R'_1$ is methyl and preferably each is hydrogen, each of $R_2$ and $R'_2$ is $-CN$ or $-COOR'''$, R''' is unsubstituted $C_1$-$C_4$-alkyl and each of $R_3$ and $R'_3$ is methyl or ethyl and preferably hydrogen.

7. A process according to Claim 1 for the production of a compound of the formula (I), wherein Q is $-COCl$, $R_1$ and $R_3$ are hydrogen, and $R_2$ is carboxylic acid $C_1$-$C_4$-alkyl ester or cyano.

8. A process according to Claim 1, wherein the compound of the formula (III) is $PdCl_2$, palladium acetylacetonate and, in particular, palladium acetate.

9. A process according to Claim 1, wherein the reaction is carried out in the temperature range from 0 to 200° C, in particular from 90 to 150° C, and in the presence of an organic solvent which is inert to the reaction components.

10. A process according to Claim 9, wherein the solvent is anisole, xylene or toluene.

11. A process according to Claim 1, wherein the base is a trialkylamine containing 4 to 12 carbon atoms in each of the alkyl moieties.

12. A process according to Claim 1, wherein 0.0001 to 20 mol-% of catalyst is used, based on the compound of formula (III).

13. A vinylstilbene compound of the formula:

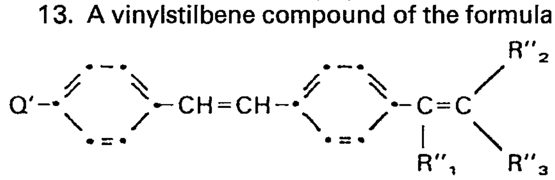

wherein Q' is $-COCl$ or

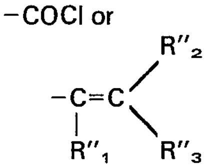

with the proviso that, if Q' is $-COCl$, $R''_1$ and $R''_3$ are hydrogen and $R''_2$ is $-COOCH_3$, $-COOC_2H_5$ or $-CN$, and, if Q' is

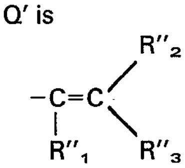

$R''_1$ and $R''_3$ are cyano and $R''_2$ is $-(CH_2)_2CN$, $-COC_2H_5$,

$-COO(CH_2)_2CN$, $-COOCH_2-$ 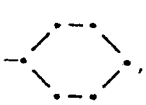,

$-COOCH_2-$ 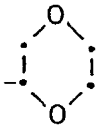 or $-COOCH_2-$ 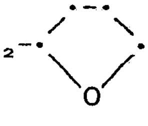

14. A method of whitening man-made, regenerated man-made and natural high molecular weight organic materials, which method comprises incorporating in or applying to the surface of said materials a vinylstilbene compound of the formula as defined in Claim 13, wherein Q' is

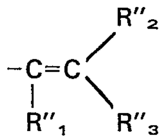

15. A method according to Claim 14, wherein the amount of fluorescent whitening agent employed is 0.001 to 2%, preferably 0.01 to 0.5%, based on the weight of the material to be whitened.

16. The method of Claim 15 for whitening polyester.

17. A method according to Claim 16 which comprises treating polyester fabrics with the respective fluorescent whitening agent by the exhaust or pad-heat process.

## Revendications

1. Procédé pour la préparation de composés vinylstilbéniques ayant la formule (IX):

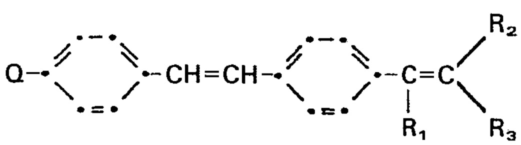 (IX)

dans laquelle Q représente le groupement $-COX$ ou

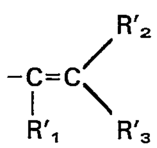 et dans laquelle $R_1$ et $R'_1$, indépendamment l'un de l'autre, sont des atomes d'hydrogène, des groupes cyano, alkyle ou des groupes carboxyle estérifiés non chromophores. $R_2$ et $R'_2$, indépendamment l'un de l'autre, sont des atomes d'hydrogène, des groupes alkyle ou alcényle portant éventuellement des substituants non chromophores, ou bien des subtituants non chromophores du deuxième ordre, $R_3$ et $R'_3$, indépendamment l'un de l'autre, sont des atomes d'hydrogène, des groupes cyano ou des groupes alkyle ou alcényle portant éventuellement des substituants non chromophores, et X est un atome de chlore, de brome ou d'iode, ou bien $R_1$ représente ensemble avec $R_3$ ou bien $R'_1$ représente ensemble avec $R'_3$ un pont alkylène, tout au plus un des symboles $R_2$ et $R_3$ et tout au plus un des symboles $R'_2$ et $R'_3$ étant des atomes d'hydrogène et où, si Q est $-COX$, $R_1$ et $R_3$ sont des atomes d'hydrogène et $R_2$ est un ester alkylique en $C_{1-4}$ d'acide carboxylique ou un groupe cyano, caractérisé par le fait qu'on fait réagir 1 mol-Eq d'un composé de formule (II):

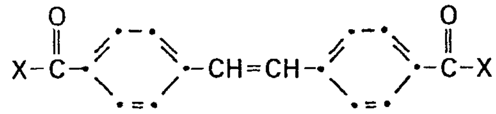 (II)

dans laquelle X a la signification donnée ci-dessus, en présence d'une base et en ajoutant du palladium métallique ou des composés de palladium qui, dans les conditions de la réaction, donnent des composés de palladium(O) labiles sans phosphore, comme catalyseurs, avec 1 mol-Eq d'un composé de formule (III) ou avec 1 mol-Eq de chacun des composés des formules (III) et (IV):

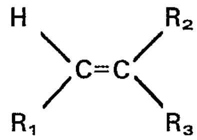 (III) 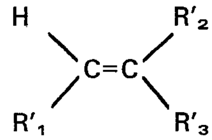 (IV)

dans lesquelles $R_1$, $R_2$ et $R_3$, ou bien $R'_1$, $R'_2$ et $R'_3$, ont les significations données ci-dessus.

2. Procédé selon la revendication 1, caractérisé par le fait qu'on utilise un composé de formule (II), dans lequel X représente un atome de chlore.

3. Procédé selon la revendication 1, caractérisé par le fait qu'on prépare un composé de formule (I), dans lequel Q représente le groupement:

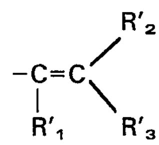

et dans lequel $R_1$ et $R'_1$, indépendamment l'un de l'autre, sont des atomes d'hydrogène, des groupes cyano, alkyle en $C_{1-6}$ ou $-COOR'''$, $R_2$ et $R'_2$, indépendamment l'un de l'autre, sont des atomes d'hydrogène ou des groupes alkyle en $C_{1-6}$ ou alcényle en $C_{2-4}$, $-CN$, $-CF_3$, éventuellement substitués par des radicaux alcoxy en $C_{1-4}$ ou alcoxycarbonyle en $C_{2-5}$, ou bien des groupes:

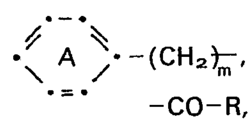

$-CO-R$, $-CO-NR'R''$ ou $-COOR'''$,

$R_3$ et $R'_3$, indépendamment l'un de l'autre, sont des atomes d'hydrogène, des groupes cyano ou des groupes alkyle en $C_{1-6}$ éventuellement substitués par des radicaux alcoxy en $C_{1-4}$ ou alcoxycarbonyle en $C_{2-5}$, ou bien $R_1$ et $R_3$ ou $R'_1$ et $R'_3$ représentent ensemble $-(CH_2)_3-$ ou $-(CH_2)_4-$, tout au plus un des $R_2$ et $R_3$ et tout au plus un des $R'_2$ et $R'_3$ étant des atomes d'hydrogène; R est un groupe méthyle, alkyle en $C_{2-6}$ éventuellement substitué par un radical alcoxy en $C_{1-4}$, alcoxyalcoxy en $C_{4-6}$, un reste de formule:

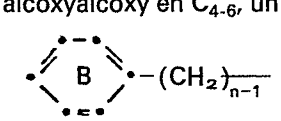 ou naphtyle,

R' est un atome d'hydrogène, un groupe méthyle, un groupe alkyle en $C_{2-6}$, éventuellement substitué par un radical alcoxy en $C_{1-4}$, alcoxyalcoxy en $C_{4-6}$

ou di(alkyl en $C_{1-4}$)-amino, un groupe cyclohexyle éventuellement méthylé ou éthylé ou bien un reste de formule:

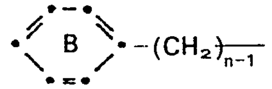

R″ est un atome d'hydrogène, un groupe méthyle ou alkyle en $C_{2-6}$ éventuellement substitué par un radical alcoxy en $C_{1-4}$ ou alcoxyalcoxy en $C_{4-6}$, R‴ est un groupe méthyle, alkyle en $C_{2-6}$ éventuellement substitué par un radical alcoxy en $C_{1-4}$ ou alcoxyalcoxy en $C_{3-6}$, un groupe cyclohexyle éventuellement méthylé et/ou éthylé ou bien un reste de formule:

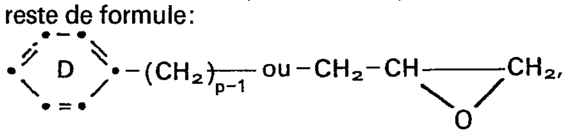

m est le nombre 1 ou 2, et n et p, indépendamment l'un de l'autre, sont les nombres 1, 2 ou 3, les noyaux A, B et D pouvant être mono- ou disubstitués par des radicaux alkyle en $C_{1-3}$ et/ou des atomes d'halogène, et les noyaux B et D, quand n et p représentent chacun le nombre 1, pouvant être trisubstitués par des radicaux alkyle en $C_{1-3}$ et/ou des atomes d'halogène, ou bien monosubstitués, ou disubstitués par des radicaux alcoxy en $C_{1-3}$.

4. Procédé selon la revendication 3, caractérisé par le fait qu'on prépare un composé de formule (I), dans lequel Q représente le groupement:

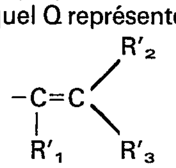

et dans lequel $R_1$ et $R'_1$, indépendamment l'un de l'autre, sont des atomes d'hydrogène, des groupes $-CN$ ou alkyle en $C_{1-4}$; $R_2$ et $R'_2$, indépendamment l'un de l'autre, sont des groupes alcényle en $C_{2-4}$, $-CN$, $-CONR'R''$, $-COOR'''$ ou $-CO-R$, et $R_3$ et $R'_3$, indépendamment l'un de l'autre, sont des atomes d'hydrogène, des groupes $-CN$ ou alkyle en $C_{1-4}$, tout au plus un des $R_2$ et $R_3$ et tout au plus un des $R'_2$ et $R'_3$ étant des atomes d'hydrogène; R est un groupe méthyle, alkyle en $C_{2-6}$, naphtyle, éventuellement substitués par des radicaux alcoxy en $C_{1-4}$ ou alcoxyalcoxy en $C_{4-6}$, ou bien un groupe phényle éventuellement subtitué par un atome d'halogène, un groupe alkyle en $C_{1-3}$ et/ou alcoxy en $C_{1-3}$; R′ et R″ sont chacun un groupe méthyle; R‴ est un groupe méthyle, alkyle en $C_{2-6}$ éventuellement subtitué par les radicaux alcoxy en $C_{1-4}$ ou alcoxyalcoxy en $C_{3-6}$; m est le nombre 1; n et p, indépendamment l'un de l'autre, sont le nombre 2 et, en particulier, le nombre 1.

5. Procédé selon la revendication 3, caractérisé par le fait qu'on prépare un composé de formule (I), dans lequel Q représente le groupement:

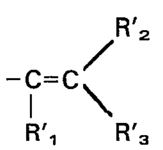

et dans lequel $R_1$ et $R'_1$ ou $R_3$ et $R'_3$ sont chacun un atome d'hydrogène, un groupe $-CN$ ou alkyle en $C_{1-4}$; $R_2$ et $R'_2$ sont chacun un groupe alcényle en $C_{2-4}$, $-CN$, $-CONR'R''$, $-COOR'''$ ou $-CO-R$, tout au plus un des $R_2$ et $R_3$ et tout au plus un des $R'_2$ et $R'_3$ représentant des atomes d'hydrogène; R est un groupe alkyle en $C_{1-6}$ ou un groupe phényle éventuellement susbtitué par un atome d'halogène, des radicaux alkyle en $C_{1-3}$ ou alcoxy en $C_{1-3}$, en particulier un groupe alkyle en $C_{1-4}$ non substitué ou bien un groupe phényle éventuellement monosubstitué par un atome de chlore, un radical méthyle ou méthoxy; R′ et R″ sont chacun un groupe méthyle, et R‴ est un groupe méthyle ou un groupe alkyle en $C_{2-6}$ éventuellement substitué par des radicaux alcoxy en $C_{1-4}$ ou alcoxyalcoxy en $C_{3-6}$, en particulier un groupe alkyle en $C_{1-4}$ non substitué.

6. Procédé selon la revendication 3, caractérisé par le fait qu'on prépare un composé de formule (I), dans lequel Q représente le groupement:

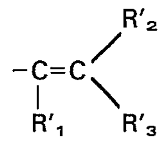

et dans lequel $R_1$ et $R'_1$ sont chacun un groupe méthyle et, en particulier, sont chacun un atome d'hydrogène; $R_2$ et $R'_2$ sont chacun un groupe $-CN$ ou $-COOR'''$; R‴ est un groupe alkyle ayant 1 à 4 atomes de carbone non substitué, et $R_3$ et $R'_3$ sont chacun un groupe méthyle ou éthyle et, en particulier, un atome d'hydrogène.

7. Procédé selon la revendication 1, caractérisé par le fait qu'on prépare un composé de formule (I), dans lequel Q représente le groupe $-COCl$, et $R_1$ et $R_3$ sont des atomes d'hyrogène, et $R_2$ est un ester alkylique en $C_{1-4}$ d'acide carboxylique ou un groupe cyano.

8. Procédé selon la revendication 1, caractérisé par le fait qu'on utilise, comme composé de formule (III), $PdCl_2$, l'acétylacétonate de palladium et, en particulier, l'acétate de palladium.

9. Procédé selon la revendication 1, caractérisé par le fait qu'on effectue la réaction à une température comprise entre 0 et 200° C, en particulier entre 90 et 150° C, et en présence d'un solvant organique inerte vis-à-vis des réactifs.

10. Procédé selon la revendication 9, caractérisé par le fait qu'on utilise, comme solvants, l'anisol, le xylène ou le toluène.

11. Procédé selon la revendication 1, caractérisé par le fait qu'on utilise, comme base, une trialkylamine dont les restes alkyle ont chacun 4 à 12 atomes de carbone.

12. Procédé selon la revendication 1, caractérisé par le fait qu'on utilie le catalyseur à raison de 0,0001 à 20% en mole par rapport au composé de formule (II).

13. Composés vinylstilbéniques de formule:

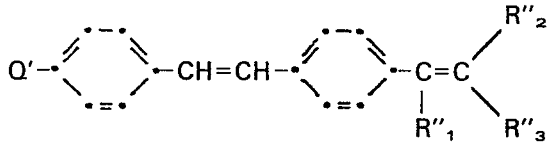

dans laquelle Q' est un groupe −COCl ou

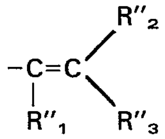

étant donné que, dans le cas où Q' est −COCl, R''₁ et R''₃ sont des atomes d'hydrogène, et R''₂ est un groupe $-COOCH_3$, $-COOC_2H_5$ ou −CN et, dans le cas où Q' est:

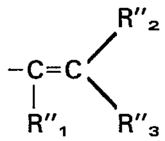

$R''_1$ et $R''_3$ sont des atomes d'hydrogène, et $R''_2$ représente:

$-COO(CH_2)_2CN$, $-COOCH_2-$ 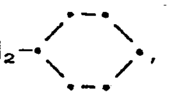,

$-COOCH_2-$ 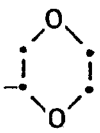 ou $-COOCH_2-$ 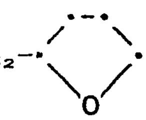

14. Procédé pour l'azurage optique de matières organiques à haut poids moléculaire, synthétiques, semi-synthétiques et naturelles, caractérisé par le fait qu'on incorpore aux matières à azurer optiquement des composés vinylstilbéniques ayant la formule définie dans la revendication 13, dans lesquels Q' est:

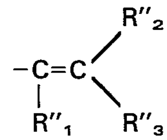

ou bien qu'on applique ces composés sur la surface des matières en question.

15. Procédé selon la revendication 14, caractérisé par le fait qu'on utilise 0,001 à 2%, de préférence 0,01 à 0,5% de l'azurant, par rapport au poids de la matière à azurer.

16. Procédé selon la revendication 15 pour l'azurage optique des polyesters.

17. Procédé selon la revendication 16, caractérisé par le fait qu'on traite les tissus en fibres de polyester avec les divers azurants au moyen du procédé par épuisement ou du procédé de thermo-foulardage.